# EUROPEAN PATENT APPLICATION

(11) **EP 4 458 327 A1**
(43) Date of publication of application: **06.11.2024**
(21) Application number: 22914156.9
(22) Date of filing: 09.12.2022
(51) Int. Cl.: A61F 2/90

(54) **LUMEN STENT**

(30) Priority: 31.12.2021 CN 202111677102; 31.12.2021 CN 202111672621
(71) Applicant: Lifetech Scientific (Shenzhen) Co., Ltd., Shenzhen, Guangdong 518063 (CN)
(72) Inventor: MING, Tingbo, Shenzhen, Guangdong 518063 (CN); XIAO, Benhao, Shenzhen, Guangdong 518063 (CN); YANG, Wulong, Shenzhen, Guangdong 518063 (CN)
(74) Representative: Michalski Hüttermann & Partner Patentanwälte mbB
(86) International application number: PCT/CN2022/137931
(87) International publication number: WO 2023/124901

(57) **Abstract**

A lumen stent, including a mesh support structure. The mesh support structure includes a first mesh region and a second mesh region connected to the first mesh region in a circumferential direction in a naturally deployed state. The first mesh region includes a plurality of rows of intersecting units formed by a plurality of first direction support wires spaced apart and a plurality of second direction support wires spaced apart overlapping each other. The second mesh region includes at least one row of hooking units, each row of the hooking units including a plurality of hooking units axially spaced apart, and the hooking unit including a first hooking member and a second hooking member. The first hooking member and the second hooking member are mutually hooked substantially in an axial direction. The lumen stent is less prone to shortening during release and has superior flexibility.

## Description

### Technical Field

The embodiments relate to the field of medical devices and to a lumen stent.

### Background

Traditional open surgical treatment of vascular diseases such as aortic aneurysms and aortic dissection has the problems of large trauma, high mortality, long operation time, high incidence of postoperative complications, and high surgical difficulty. The use of microtrauma intervention in the treatment of vascular diseases has the advantages of less trauma, high safety, and high effectiveness, so it has been affirmed by doctors and patients and has become an important treatment method for vascular diseases. Interventional therapy refers to the use of a delivery system to implant vascular stents into the patient's vascular lesion segment. The implanted vascular stents can support the narrow occlusive segment of blood vessels or block the vascular dissection rupture through expansion, reduce the elastic retraction and remodeling of blood vessels, keep the lumen blood flow unobstructed, and prevent vascular stenosis.

Vascular stents can be divided into cut stents and braided stents according to different processes. Due to its superior flexibility, the braided stent can be used for the treatment of vascular diseases with curved channels. Currently, known braided stents include a plurality of wave coils connected, each wave coil having a plurality of spaced wave peaks and wave troughs. To provide the braided stent with a relatively stable tubular configuration, the braiding between adjacent wave coils is generally performed in such a manner that the wave peak and wave trough are all constrained to each other, and the constrained manner includes hooking the opposite wave peaks and wave troughs mutually, or performing suture connection on the opposite wave peaks and wave troughs. The mutual hooking can make a certain movement space between adjacent wave coils in the axial direction, so that the stent can better conform to the vascular cavity, but also make the stent prone to shortening during release. Constraining the opposite wave peaks and wave troughs through suture connection can solve the problem of prone to shortening of the stent, but compromise the flexibility of the stent.

### Summary

In response to the above problems, it would be desirable to provide a lumen stent that is less prone to shortening during release and has superior flexibility to conform to a variety of vascular lumen shapes.

The embodiments provide a lumen stent including a mesh support structure; the mesh support structure includes a first mesh region and a second mesh region connected to the first mesh region in a circumferential direction in a naturally deployed state; the first mesh region includes a plurality of rows of intersecting units formed by a plurality of first direction support wires spaced apart and a plurality of second direction support wires spaced apart overlapping each other; the second mesh region includes at least one row of hooking units, each row of the hooking units including one or more hooking units arranged axially, and the hooking unit including a first hooking member and a second hooking member; the first hooking member and the second hooking member in at least portion of the hooking units are mutually hooked substantially in an axial direction, or the first hooking member and the second hooking member in at least portion of the hooking units are mutually separated substantially in the axial direction to form a gap, or the first hooking member and the second hooking member in at least portion of the hooking units abut, but do not hook.

In one embodiment, the lumen stent includes a main body stent, and the main body stent includes a proximal segment, a distal segment, and an intermediate segment positioned between the proximal segment and the distal segment, the intermediate segment including an intermediate unit and at least one mesh support structure, and at least a portion of the mesh support structure and an outer surface of the intermediate unit forming a gap in a radial direction; the main body stent has an inner cavity extending through the proximal segment, the distal segment, and the intermediate unit, the gap being in communication with the inner cavity.

In one embodiment, the mesh support structure includes two second mesh regions in a circumferential direction, the two second mesh regions connecting to two sides of the first mesh region, respectively; the mesh support structure is connected to the intermediate unit through the second mesh regions on two sides.

In one embodiment, the first hooking member includes a wave trough and two wave rods connected to the wave trough, and the second hooking member includes a wave peak and two wave rods connected to the wave peak; the first hooking member and the second hooking member are mutually hooked such that the wave trough of the first hooking member and the wave peak of the second hooking member are relatively movable in the axial direction.

In one embodiment, the mesh support structure further includes at least one connector connected to the second mesh region, and the mesh support structure is connected to the intermediate unit through the connector.

In one embodiment, the connector is connected to one first hooking member and one second hooking member, and the connector includes a wave towards a circumferential direction; the wave shares one wave rod with the first hooking member and one wave rod with the second hooking members.

In one embodiment, the connector is connected to one first hooking member and one second hooking member, and the connector includes two waists and a bottom edge connecting the two waists; one waist is formed by extending one wave rod of the first hooking member, the other waist is formed by extending one wave rod of the second hooking member, and the two waists slidably move relative to each other at an intersection.

In one embodiment, the lumen stent further includes at least one support unit; the support unit is provided at a peripheral edge of the mesh support structure.

In one embodiment, the second mesh region includes a proximal region, an intermediate region, and a distal region from a proximal end to a distal end; a hooking gap of at least one hooking unit in the intermediate region is greater than a hooking gap of a hooking unit in the proximal region and greater than a hooking gap of a hooking unit in the distal region.

In one embodiment, the distal end of the first hooking member and the proximal end of the second hooking member are mutually hooked, and the distal end of the first hooking member and/or the proximal end of the second hooking member are bent inward.

A lumen stent has a tubular shape in a naturally deployed state, and the lumen stent includes a first mesh region and a second mesh region connected to the first mesh region in a circumferential direction in a naturally deployed state; the first mesh region includes a plurality of rows of intersecting units formed by a plurality of first direction support wires spaced apart and a plurality of second direction support wires spaced apart overlapping each other; the second mesh region includes at least one row of hooking units, each row of the hooking units including one or more hooking units arranged axially, and the hooking unit including a first hooking member and a second hooking member; the first hooking member and the second hooking member in at least portion of the hooking units are mutually hooked substantially in an axial direction, or the first hooking member and the second hooking member in at least portion of the hooking units are mutually separated substantially in the axial direction to form a gap, or the first hooking member and the second hooking member in at least portion of the hooking units are abut, but do not hook.

In one embodiment, the lumen stent includes a first mesh region and two second mesh regions in a circumferential direction, the two second mesh regions connecting two sides of the first mesh region, respectively.

In one embodiment, the first direction support wire extends to a first side for connection with the second hooking member, and the first direction support wire extends to a second side for connection with the first hooking member, the second direction support wire extends to a first side for connection with the first hooking member, and the second direction support wire extends to a second side for connection with the second hooking member.

In one embodiment, the first hooking member includes a wave trough and two wave rods connected to the wave trough, the second hooking member includes a wave peak and two wave rods connected to the wave peak, and the first hooking member and the second hooking member in at least portion of the hooking units are mutually hooked such that the wave trough of the first hooking member and the wave peak of the second hooking member are relatively movable in the axial direction.

In one embodiment, the lumen stent further includes a third mesh region in the circumferential direction; two sides of the third mesh region are each connected to one second mesh region; or two sides of the third mesh region are each connected to one first mesh region; or two sides of the third mesh region are each connected to the first mesh region and the second mesh region, respectively.

In one embodiment, the third mesh region includes one or more waveform unit groups spaced apart in an axial direction, and the waveform unit group includes two waveform units mutually hooked in the axial direction.

In one embodiment, at least one set of two waveform units mutually hooked in the axial direction in the third mesh region has a hooking gap smaller than a hooking gap of the hooking units in the second mesh region.

In one embodiment, the second mesh region includes a proximal region, an intermediate region, and a distal region from a proximal end to a distal end; a hooking gap of at least one hooking unit in the intermediate region is greater than a hooking gap of a hooking unit in the proximal region and greater than a hooking gap of a hooking unit in the distal region.

In one embodiment, the distal end of the first hooking member and the proximal end of the second hooking member are mutually hooked, and the distal end of the first hooking member and/or the proximal end of the second hooking member are bent inward.

A stent system includes the lumen stent according to any one of the above and a covered stent connected to the lumen stent, the lumen stent being sleeved outside the covered stent, and the lumen stent forming a gap with an outer surface of the covered stent in a radial direction.

The second mesh region of the lumen stent provided by the embodiments has at least superior flexibility because the second mesh region includes a hooking unit, and at least some of the hooking units can slide relatively between a first hooking member and a second hooking member hooked on each other, and the first mesh region includes a plurality of rows of intersecting units formed by a plurality of spaced-apart first direction support wires and a plurality of spaced-apart second direction support wires overlapping each other, and the first mesh region can limit the shortening of the second mesh structure to a certain extent by being connected to the second mesh region. As a result, the lumen stent of the embodiments is less prone to shortening during release and has superior flexibility to conform to a variety of vascular lumen shapes.

### Brief Description of the Drawings

Fig. 1 is an overall structural diagram of a lumen stent according to an embodiment;
Fig. 2 is a cross-sectional view of an intermediate main body covering film in the lumen stent shown in Fig. 1;
Fig. 3 is a diagram after a connection between a second intermediate covering film and a branch stent in a lumen stent shown in Fig. 1;
Fig. 4 is a structural diagram of a mesh support structure in a lumen stent shown in Fig. 1;
Fig. 5 is an enlarged partial view of a first mesh region in Fig. 4;
Fig. 6 is a diagram of a first hooking state of a hooking unit in Fig. 4;
Fig. 7 is a diagram of a second hooking state of a hooking unit in Fig. 4;
Fig. 8 is a diagram of a third hooking state of a hooking unit in Fig. 4;
Fig. 9 is a diagram showing that a first hooking member and a second hooking member of a hooking unit in Fig. 4 are tilted outward;
Fig. 10 is a structural diagram of a mesh support structure according to another embodiment;
Fig. 11 is a waveform angle diagram of a first hooking member and a second hooking member;
Fig. 12 is a structural diagram of a hooking unit according to another embodiment;
Fig. 13 is a structural diagram of a connector in Fig. 1;
Fig. 14 is a structural diagram of a mesh support structure according to another embodiment;
Fig. 15 is a structural diagram of a connector in Fig. 14;
Fig. 16 is a structural diagram of a mesh support structure according to yet another embodiment;
Fig. 17 is a structural diagram of a lumen stent according to another embodiment;
Fig. 18 is an expansion view of a mesh support structure in Fig. 17;
Fig. 19 is a diagram of a lumen stent of Fig. 17 implanted in a stenotic vessel;
Fig. 20 is a diagram of a lumen stent exclusion tumor body of Fig. 17;
Fig. 21 is a structural diagram of a lumen stent according to yet another embodiment;

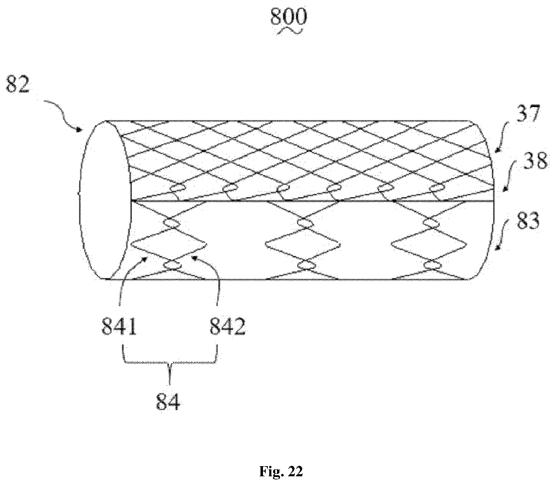

Fig. 22 is a structural diagram of a lumen stent according to still another embodiment;
Fig. 23 is a diagram of a lumen stent of Fig. 22 implanted in an aortic arch;
Fig. 24 is a structural diagram of a stent system according to the embodiments; and
Fig. 25 is an exploded diagram of a main body covering film of Fig. 24.

### Detailed Description of the Embodiments

For a better understanding of the concepts, reference will now be made in detail to the embodiments, examples of which are illustrated in the accompanying drawings.

For ease of description, spatially relative terms, such as "inner", "outer", "inside", "outside", "below", "under", "above", "over", and the like, may be used herein to describe one element or feature's relationship to another element (s) or feature (s) as illustrated in the drawings. Such spatially relative terms are intended to encompass different orientations of the device in use or operation in addition to the orientation depicted in the drawings. For example, if the device in the drawings is turned over, elements described as "below" or "under" other elements or features would then be oriented "above" or "over" the other elements or features. Thus, the exemplary term "under" can include both an upper and a lower orientation. The device may be otherwise oriented (rotated 90 degrees or in other directions) and the spatially relative descriptors used herein interpreted accordingly.

To describe the structure of the embodiments more clearly, the terms "proximal" and "distal" are defined herein as conventional terms used in the art of interventional medicine. For example, "distal" refers to the end from which blood flows, and "proximal" refers to the end into which blood flows, for example, after stent implantation, blood flows from the proximal end toward the distal end of the stent; "axial" refers to its lengthwise direction, and "radial" refers to a direction perpendicular to the "axial" direction.

A "wave coil" (also referred to as a waveform ring) in the embodiments is a closed ring structure, and a "waveform unit" is an arc structure. "Wave coil" and "waveform unit" are made by weaving or cutting metal elastic material or polymer material. The metallic elastic materials include known materials in implantable medical devices or combinations of various biocompatible materials, such as alloys of two or more single metals of cobalt, chromium, nickel, titanium, magnesium, iron, as well as 316L stainless steel, nickel-titanium-tantalum, or other biocompatible metallic elastic materials. Polymer materials include materials with biocompatibility such as polylactic acid. Both the "wave coil" and the "waveform unit" have the radial expansion capability, which can realize radial contraction under the action of external force, and recover to the original shape by self-expansion or mechanical expansion (such as expansion through balloon expansion) after the external force is withdrawn, and maintain the original shape, so that they can abut against the inner wall of the lumen through their radial supporting force after the lumen is implanted. The waveforms of the waves in the "wave coil" and "waveform unit" are not limited, and include a Z-shaped wave, an M-shaped wave, a V-shaped wave, a sine wave, etc. The "wave coil" and the "waveform unit" each include a plurality of wave peaks (also called proximal apexes), a plurality of wave troughs (also called distal apexes), and wave rods connecting adjacent wave peaks and wave troughs. One vertex (proximal vertex or distal vertex) and two wave rods connected to the vertex form one wave.

The "covering film" in the embodiments can insulate liquid to a certain extent and can be made of high polymer materials with good biocompatibility, such as poly tetra fluoroethylene (abbreviated as PTFE) and polyethylene terephthalate (abbreviated as PET).

### Embodiment 1

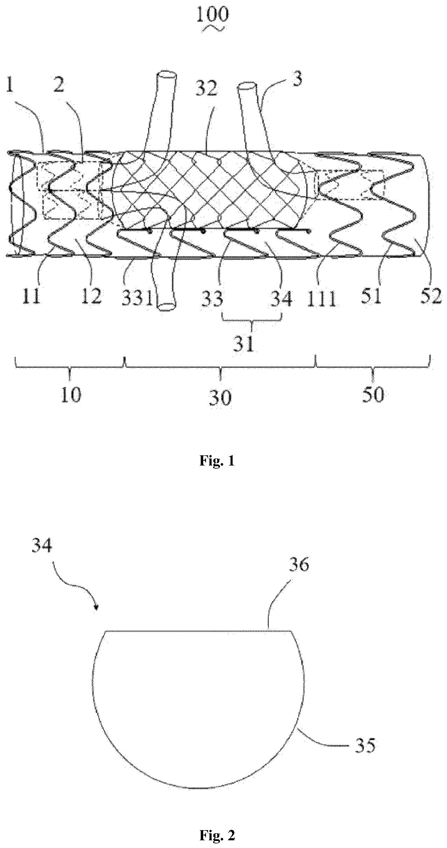

As shown in Fig. 1, the lumen stent 100 of the embodiment has a hollow tubular structure with openings at two ends and includes a main body stent 1 and a branch stent 2 provided in the main body stent 1, the inner cavity of the main body stent 1 and the inner cavity of the branch stent 2 being in communication.

The main body stent 1 includes a main body support part and a main body covering film, and the main body covering film may be provided on an inner surface and/or an outer surface of the main body support part, or the main body covering film may be provided on a portion of the inner surface and a portion of the outer surface of the main body support part.

The main body stent 1 may be axially divided into a proximal segment 10, a distal segment 50, and an intermediate segment 30 between the proximal segment 10 and the distal segment 50.

The proximal segment 10 includes a tubular proximal support part 11 and a proximal main body covering film 12, the proximal main body covering film 12 may be applied to the inside and/or outside of the proximal support part 11 by sewing, bonding, heat-fusing, or the like. The proximal support part 11 includes a plurality of axially spaced main body wave coils 111.

The distal segment 50 includes a tubular distal support part 51 and a distal main body covering film 52, the distal main body covering film 52 may also be applied to the inside and/or outside of the distal support part 51 by stitching, adhesive, heat staking, or the like. The distal support part 51 includes a plurality of axially spaced main body wave coils 111.

The intermediate segment 30 includes an intermediate unit 31 and a mesh support structure 32. The intermediate unit 31 includes an intermediate main body covering film 34 and an intermediate support part 33 (which may be omitted in other embodiments). The inner cavity defined by the closure of the intermediate main body covering film 34 communicates with the inner cavity defined by the closure of the proximal main body covering film 12 and the inner cavity defined by the closure of the distal main body covering film 52. The two sides of the mesh support structure 32 in the circumferential direction are respectively fixedly connected to the intermediate main body covering film 34 through sewing, bonding, hot melting, etc. and at least portion of the mesh support structure 32 and the outer surface of the intermediate unit 31 form a gap (or a space, a cavity) which can communicate with the inner cavity of the branch stent 2. When it is necessary to implant a bridging stent 3 for a branch vessel, the bridging stent 3 can pass through the mesh support structure 32 to communicate with the branch stent 2; in the process of reconstructing a branch, blood flow can enter the gap between the mesh support structure 32 and the intermediate unit 31 through the branch stent 2, and then enter the branch vessel from the mesh support structure 32 without a covering film, to avoid the problem of long-term ischemia of the branch vessel during the process of reconstructing the branch; for twisted lumens, such as lumens with smaller true lumens, by providing a gap between the mesh support structure 32 and the intermediate unit 31 to provide a more stable operating space intraoperatively, compression of the operating space due to compression of the intermediate unit 31 by the lumen may be avoided.

In the embodiment, the mesh support structure 32 is substantially arc-shaped, and the projection of the mesh support structure 32 on a radial plane corresponds to a central angle of less than or equal to 120 degrees so that the mesh support structure 32 has a good radial support force, and sufficient space within the intermediate unit 31 is ensured for the passage of blood flow, and at the same time the radial compression size of the intermediate segment 30 can be adapted.

Referring also to Fig. 2, in the embodiment, the intermediate main body covering film 34 includes a first intermediate covering film 35 having an arcuate sheet-like structure and a second intermediate covering film 36 connected to the first intermediate covering film 35 to form a circumferentially sealed cavity. The first intermediate covering film 35 is sewn, adhered, heat-fused, or the like on the inner side and/or the outer side of the intermediate support part 33. The intermediate support part 33 includes a plurality of intermediate waveform units 331 arranged at intervals in the axial direction. At least a portion of the mesh support structure 32 forms a gap with the outer surface of the second intermediate covering film 36. In addition, the second intermediate covering film 36 is a sheet-like structure generally on the same plane, or the second intermediate covering film 36 is an arc-shaped sheet-like structure, but the arc thereof is smaller than that of the first intermediate covering film 35 so that the gap between the second intermediate covering film 36 and the mesh support structure 32 is large enough to provide a more suitable operation space for implanting the bridging stent 3.

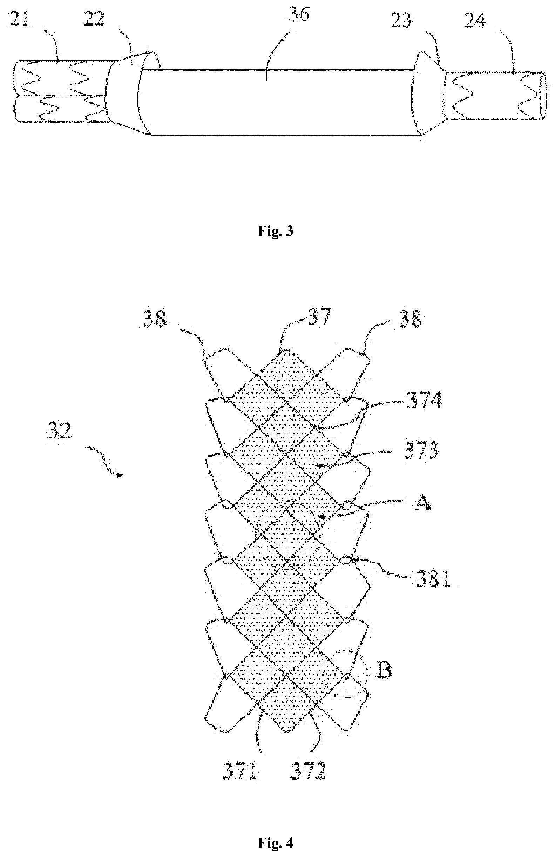

Referring to Fig. 3, the branch stent 2 may include a branch support part and a tubular branch covering film. The branch covering film may be applied to the inside and/or outside of the branch support part by sewing, bonding, heat staking, etc.

The branch stent 2 includes a proximal tube body 21 disposed within the proximal segment 10, and a distal tube body 24 disposed within the distal segment 50. The proximal tube body 21 communicates with the inner cavity of the proximal segment 10 and with the gap formed by the outer surface of the intermediate unit 31 and the mesh support structure 32. The distal tube body 24 communicates with the inner cavity of the distal segment 50 and with the gap formed by the outer surface of the intermediate unit 31 and the mesh support structure 32. The proximal tube body 21 and the distal tube body 24 may be provided with only 0 or more pieces, respectively. For example, when it is applied to the aortic arch, two proximal tube bodies 21 and one distal tube body 24 may be provided; or, one proximal tube body 21 and two distal tube bodies 24 may be provided, or all of them may be provided at the proximal or distal ends, and the specific arrangement and selection may be made according to the number and position of bridging stents 3 to be accessed so that they can meet the requirements of emergency surgery in clinical use. The presence or absence and the number of the proximal tube body 21 and the distal tube body 24 can be set correspondingly according to the actual situations that may occur, to design the lumen stent 100 of various sizes for optimal selection in clinical emergency, and thus the specific embodiments thereof are not limited to the above-mentioned exemplary embodiments.

In the embodiment, the proximal tube body 21 is connected to the second intermediate covering film 36 via the proximal transition segment 22, the distal tube body 24 is connected to the second intermediate covering film 36 via the distal transition segment 23, the proximal transition segment 22 communicates with the proximal tube body 21, the distal transition segment 23 communicates with the distal tube body 24, the distal end of the proximal transition segment 22 has a proximal outer opening communicating with the gap, the proximal end of the distal transition segment 23 has a distal outer opening communicating with the gap, and the proximal outer opening of the proximal transition segment 22 is arranged opposite to the distal outer opening of the distal transition segment 23. Both the proximal transition segment 22 and the distal transition segment 23 may be flared with the reduced end of the flared proximal transition segment 22 facing the proximal end of the body and the reduced end of the flared distal transition segment 23 facing the distal end of the body. The bridging stent 3 is accessible through the proximal outer opening and the distal outer opening during surgery. The enlarged ends of the proximal transition segment 22 and the distal transition segment 23 are both towards the intermediate segment 30, such as towards the mesh support structure 32, and gradually contract away from the mesh support structure 32, to provide a relatively large implantation guiding path for the bridging stent 3 at the initial stage of implantation, to facilitate the rapid implantation of the bridging stent 3, and gradually contract the implantation path thereof, thereby facilitating the more accurate and stable implantation of the bridging stent 3; after the bridging stent 3 is implanted into the branch stent 2, a certain fixation is performed due to the mesh support structure 32; it can thus be avoided that the bridging stent 3 swings with the blood flow, so that the bridging stent 3 is relatively more robust. In addition, the branch stent 2 may be located on the same side of the lumen stent 100 as the mesh support structure 32 to reduce the path length of the bridging stent 3 into the branch stent 2 to facilitate implantation of the bridging stent.

In other embodiments, the above-mentioned branch stent 2 may be omitted, but the intermediate unit 31 is fenestrated to provide one or more windows through which the bridging stent 3 communicates with the main body stent 1. Alternatively, in other embodiments, the lumen stent 100 may include both the branch stent 2 and one or more windows on the intermediate unit 31. However, it should be noted that if the branch stent 2 is omitted, both ends of the intermediate unit 31 should be sealingly connected to the proximal main body covering film 12 and the distal main body covering film 52 as far as possible, for example, to the proximal segment 10 via a proximal sealing film (not shown) and to the distal segment 50 via a distal sealing film (not shown).

It can be appreciated that the proximal support part 11, the distal support part 51, and the intermediate support part 33 are all part of the main body support part and may be the same or different in construction, shape, size, and material from which they are made. The proximal main body covering film 12, the distal main body covering film 52, and the intermediate main body covering film 34 described above are all part of a main body covering film, may be of a one-piece construction or a split-piece construction (e.g. may be formed by splicing multiple covering films). In addition, the thickness of the proximal main body covering film 12, the distal main body covering film 52, and the intermediate main body covering film 34 may be the same or different materials.

The mesh support structure 32 of the embodiment is formed by integrally weaving support wires. In other embodiments, the mesh support structure 32 may be formed by separately weaving and splicing support wires or may be formed by cutting.

Referring to Fig. 4, in the embodiment, the mesh support structure 32 includes a first mesh region 37 and a second mesh region 38. The first mesh region 37 and the second mesh region 38 are spaced apart in the circumferential direction and are connected to each other. Each of the first mesh region 37 and the second mesh region 38 may be radially contracted by an external force and self-expand or be mechanically expanded (e.g. by balloon inflation) to return to and retain the original shape after the external force is removed.

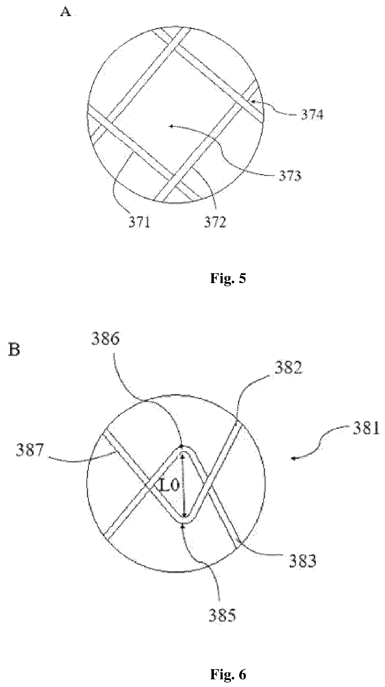

The first mesh region 37 includes a plurality of spaced apart first direction support wires 371 and a plurality of spaced apart second direction support wires 372. The first direction support wire 371 extends generally in a first direction and the second direction support wire 372 extends generally in a second direction. The first direction support wires 371 and the second direction support wires 372 are overlapped with each other to form a plurality of rows of quadrangular meshes 373 and a plurality of rows of intersecting units 374. Each row of the quadrangular meshes 373 includes a plurality of quadrangular meshes 373 arranged substantially in an axial direction, and adjacent quadrangular meshes 373 are connected to each other. Each row of intersecting units 374 includes a plurality of intersecting units 374 spaced apart substantially in an axial direction. Referring also to Fig. 5, the quadrangular mesh 373 is generally diamond-shaped and may have other shapes such as square, rectangular, etc. Four intersecting units 374 are correspondingly provided at the positions of the four corners of the quadrangular mesh 373. Each intersecting unit 374 includes a crossing point at which the first direction support wire 371 and the second direction support wire 372 overlap with each other, and at which the first direction support wire 371 and the second direction support wire 372 are relatively slidable. The first direction support wire 371 in the partial intersecting unit 374 is located outside the second direction support wire 372, and the first direction support wire 371 in the partial intersecting unit 374 is located inside the second direction support wire 372. In other embodiments, the first direction support wires 371 in the first mesh region 37 are all located outside of the second direction support wires 372, or the first direction support wires 371 are all located inside of the second direction support wires 372.

Referring also to Figs. 6-8a, the second mesh region 38 includes at least one row of hooking units 381, the row of hooking units 381 includes a plurality of hooking units 381 arranged axially spaced apart, each hooking unit 381 including a first hooking member 382 and a second hooking member 383. The first hooking member 382 and the second hooking member 383 are both part of the support wire; the first hooking member 382 includes a wave rising in the distal direction, such as including a wave trough 385 (also referred to as a distal apex) and two wave rods 387 connected to the wave trough 385; the second hooking member 383 includes a wave rising in the proximal direction, i.e. includes a wave peak 386 (also called proximal apex) and two wave rods 387 connected to the wave peak 386. In the embodiment, the first hooking member 382 and the second hooking member 383 of each hooking unit 381 are mutually hooked substantially in the axial direction. It should be noted that "substantially in an axial direction" herein means that the line between the distal apex of the first hooking member 382 and the proximal apex of the second hooking member is parallel to the axis of the mesh support structure 32, or that the line makes an angle of less than or equal to 45 with the axis of the mesh support structure 32.

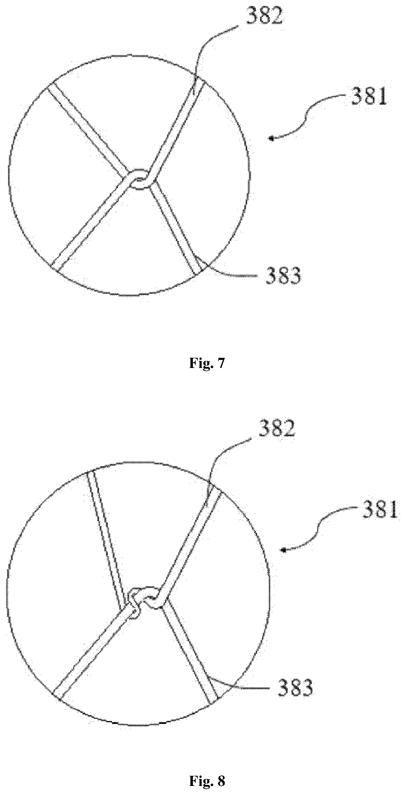

The connection state in which the first hooking member 382 and the second hooking member 383 are mutually hooked includes at least a first hooking state, a second hooking state, and a third hooking state. Referring to Fig. 6, the first hooking state is: in the naturally unfolded state, the first hooking member 382 and the second hooking member 383 hook with each other, and there is a hooking gap L0 (such as a distance apart) between the wave trough 385 of the first hooking member 382 and the wave peak 386 of the second hooking member 383, so that the first hooking member 382 can move in the proximal or distal direction, and the second hooking member 383 can also move in the proximal or distal direction, but the hooking gap L0 limits the distance the first hooking member 382 moves in the proximal direction and the second hooking member 383 moves in the distal direction. Referring to Fig. 7, the second hooking state is: in the naturally deployed state, the first hooking member 382 and the second hooking member 383 hook with each other and abut (the hooking gap L0 is 0) at the wave trough 385 of the first hooking member 382 and the wave peak 386 of the second hooking member 383, so that restrictions on the movement of the first hooking member 382 towards the proximal end and the movement of the second hooking member 383 towards the distal end. Referring to Fig. 8, the third hooking state is: in a naturally unfolded state, the first hooking member 382 and the second hooking member 383 are hooked on each other, and the wave trough 385 of the first hooking member 382 and the wave peak 386 of the second hooking member 383 are constrained to each other (for example, by welding, intertwining, intertwining with a wire such as a tantalum wire, etc.), and the first hooking member 382 and the second hooking member 383 cannot slide relatively in an axial direction. In the embodiment, the connection in which the first hooking member 382 and the second hooking member 383 are mutually hooked is either a first hooking state or a second hooking state, so that the first hooking member 382 and the second hooking member 383 in the hooking unit 381 are relatively movable in the axial direction (i.e. can be slidably connected), and the wave trough 385 of the first hooking member 382 and the wave peak 386 of the second hooking member 383 are relatively movable in the axial direction. In other embodiments, the coupling state in which the first hooking member 202 and the second hooking member 203 are mutually hooked may be one or more of the above three hooking states.

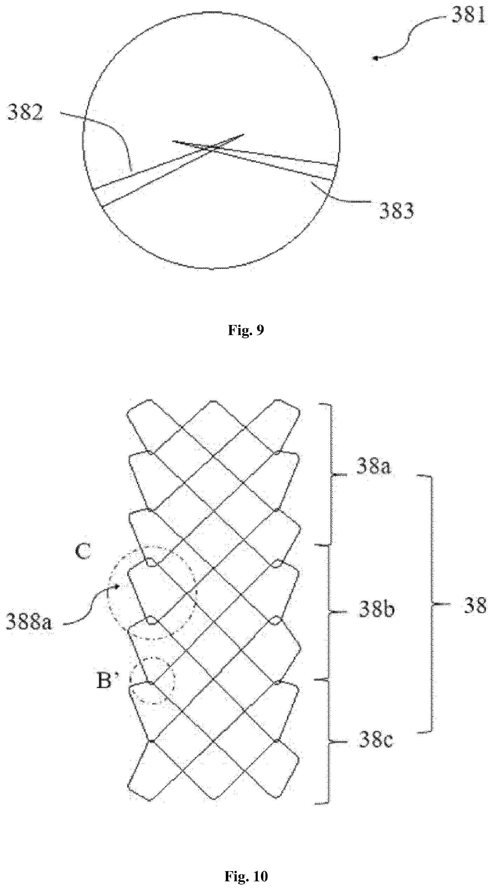

Referring to Fig. 10, the second mesh region 38 of the embodiment includes, in proximal to distal direction, a proximal region 38a, an intermediate region 38b, and a distal region 38c. In the naturally deployed state, all the hooking units 381 in the intermediate region 38b are in the first hooking state, the hooking units 381 in the proximal region 38a and the distal region 38c can be one or more of the first hooking state, the second hooking state and the third hooking state, and the hooking gaps L0 of all the hooking units 381 in the intermediate region 38b are greater than the hooking gaps L0 of the hooking units 381 in the proximal region 38a and also greater than the hooking gaps L0 of the hooking units 381 in the distal region 38c. Therefore, in the hooking unit 381 of the intermediate region 38b, there is more space for the first hooking member 382 and the second hooking member 383 to move toward both ends, respectively. When the side of the lumen stent 100 on which the mesh support structure 32 is positioned is bent outwardly, the intermediate region 38b may be stretched to a greater degree so that the lumen stent 100 may better conform to the bent lumen and better conform to the lumen inner wall. In addition, since the hooking gap L0 of the hooking unit 381 in the proximal region 38a and the distal region 38c is smaller, the first hooking member 382 and the second hooking member 383 in the hooking unit 381 are more restricted from each other, and the wave troughs 385 of the first hooking member 382 and the wave peaks 386 of the second hooking member 383 are not easily tilted or bowed outward as shown in Fig. 9, thereby reducing irritation and damage to the inner wall of the lumen by the lumen stent 100. In other embodiments, the hooking gap L0 of a portion of the hooking unit 381 (e.g. at least one hooking unit 381) in the intermediate region 38b is greater than the hooking gap L0 of the hooking unit 381 in the proximal region 38a and greater than the hooking gap L0 of the hooking units 381 in the distal region 38c, so long as the intermediate region 38b can be stretched to a greater extent.

In other embodiments, the hooking gaps L0 of the hooking units 381 in the proximal region 38a, the intermediate region 38b, and the distal region 38c may be substantially equal, and the embodiments are not limited thereto.

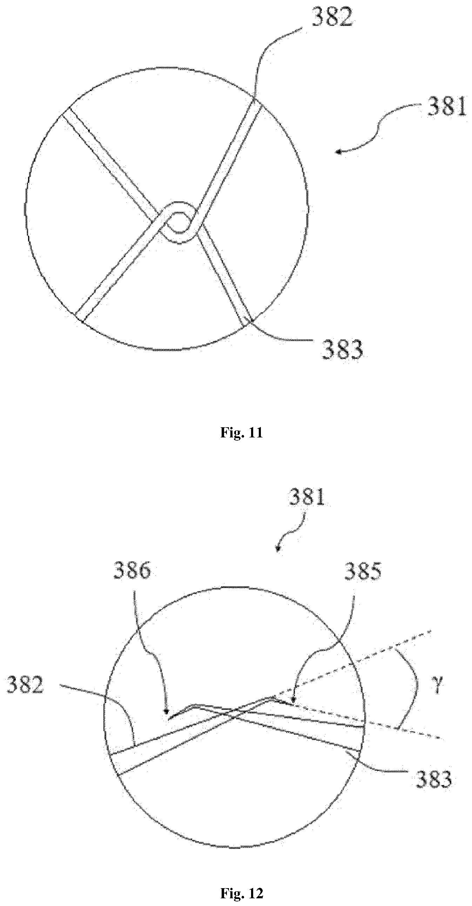

Referring to Fig. 11, to further reduce irritation and damage to the inner wall of the lumen by the lumen stent 100, the waveform angle α of the first hooking member 382 and the waveform angle β of the second hooking member 383 (i.e. an included angle between the wave rods 387) in the second mesh region 38 may be set in the range of 30° to 120°, and in other embodiments, the waveform angles α, β may be in the range of 75° to 110°. In addition, the waveform angle of the first hooking member 382 and second hooking member 383 in the intermediate region 38b may be made smaller than or equal to the waveform angle of the first hooking member 382 and second hooking member 383 in the proximal region 38a and distal region 38c to further prevent the first hooking member 382 and second hooking member 383 in the proximal region 38a and distal region 38c from lifting outward when the lumen stent 100 is bent.

Referring to Fig. 12, in other embodiments, the first hooking member 382 is bent inward at a position near its wave trough 385 (i.e. the distal end of the first hooking member 382 is bent inward), and/or the second hooking member 383 is bent inward at a position near its wave peak 386 (i.e. the proximal end of the second hooking member 383 is bent inward), the bending angle of the first hooking member 382 near its wave trough 385 may be the same as or different from the bending angle of the second hooking member 383 near its wave peak 386, and the bending angle y may range from 0 to 45°. This has at least the advantages in that, on the one hand, when the lumen stent 100 is bent, the degree to which the first hooking member 382 and the second hooking member 383 are tilted outward can be reduced, and on the other hand, it avoids that the wave peaks 386 of the first hooking member 382 and the wave peaks 386 of the second hooking member 383 are directly against the inner wall of the lumen, the relatively smooth bent portions of the first hooking member 382 and second hooking member 383 come into contact with the inner wall of the lumen, thereby further reducing the possibility of the hooking unit 381 damaging the inner wall of the lumen.

The second mesh region 38 may be connected to the intermediate unit 31 by stitching, adhesive, or the like. For example, in the embodiment, the second mesh region 38 is connected to the second intermediate covering film 36 by stitching and abuts against the intersection of the first intermediate covering film 35 and the second intermediate covering film 36. This connection is simple and reduces the possibility of damage to the covering film and the lumen inner wall by the support wires at the edge of the second mesh region 38 during use.

The first mesh region 37 of the embodiment can restrict the contraction of the second mesh structure to some extent, and the curved surface of the first mesh region 37 is smoother and less irritating to the inner wall of the lumen. The second mesh region 38 is more flexible, conforms to a variety of vessel lumen configurations, and has less recoil to reduce compression and damage to the curved lumen wall. When the bridging stent 3 needs to be implanted, the meshes of the first mesh region 37 and the second mesh region 38 can be enlarged by an external force during the process of implanting the bridging stent 3, to facilitate the bridging stent 3 to pass through the meshes, and after the bridging stent 3 is implanted, the meshes can be restored to an initial size by withdrawing the external force, to play a certain supporting and limiting role on the bridging stent 3, reducing the occurrence of the bridging stent 3 swinging with blood or heart pulsation, to ensure the stability of branch blood supply; in addition, due to the higher elongation of the first mesh region 37, it is better able to bring about the axial extension of the hooking unit 381 connected thereto (for example, the first hooking member 382 is moved proximally and the second hooking member 383 is moved distally), so that the meshes in the second mesh region 38 can be expanded sufficiently to facilitate the passage of the bridging stent 3 from the second mesh region 38.

In the embodiment, the mesh support structure 32 includes a first mesh region 37 and two second mesh region 38, the two second mesh region 38 is circumferentially spaced on either side of the first mesh region 37. In other embodiments, a plurality of first mesh regions 37 and a plurality of second mesh regions 38 may be provided, or, in other embodiments, only one first mesh region 37 and one second mesh region 38 may be provided, and the embodiments are not limited to the number of first mesh regions 37 and second mesh regions 38.

The area ratio of the first mesh region 37 to the second mesh region 38 affects the performance of the mesh support structure 32. If the area ratio is too large, the flexibility of the mesh support structure 32 will be poor, the straightening force will be large, and the elongation will be high. Too small an area ratio makes the mesh support structure 32 easier to shorten, and the mesh is less prone to deformation to facilitate the implantation of the bridging stent 3. Referring to Fig. 4, Fig. 4 is a plan view of the mesh support structure 32, the ratio of the area of the first mesh region 37 (in the figure, the mesh 373 in the first mesh region 37 is filled with wave points) to the area of the second mesh region 38 (in the figure, the mesh is not filled with wave points) of the embodiment is 1-2, so that the mesh support structure 32 has better flexibility, less straightening force, and the shape during and after the radial expansion is more stable, and the bridging stent 3 is also easy to be implanted.

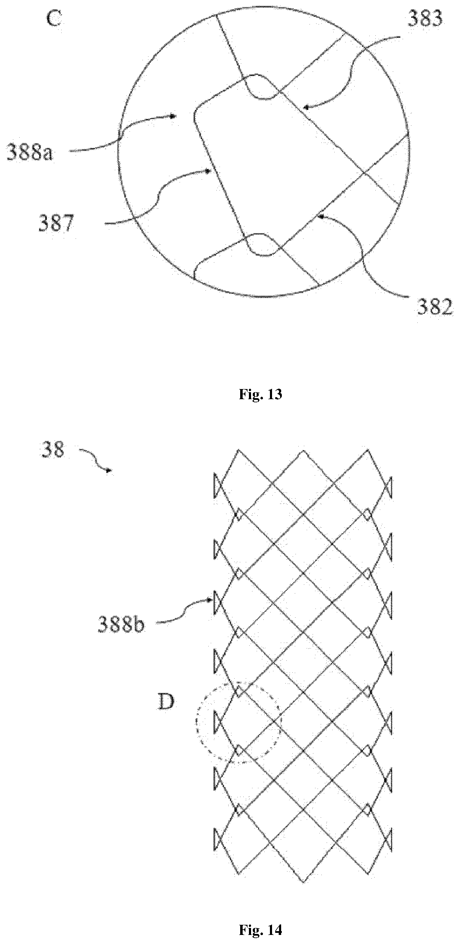

Referring also to Figs. 10 and 13, the second mesh region 38 is further provided with a plurality of connectors 388a. In the embodiment, each connector 388a is connected to a first hooking member 382 and a second hooking member 383. The connector 388a includes a wave rising toward one side in the circumferential direction, which shares a wave rod 387 with the first hooking member 382 and a wave rod 387 with the second hooking member 383. A connector 388a and first hooking member 382 and second hooking member 383 connected thereto cooperate to enclose a mesh of the second mesh region 38. The connector 388a may be fixedly connected to the intermediate unit 31 by sewing, bonding, etc. Since connector 388a has an apex projecting toward the circumferential direction, the connection can be made stronger by fixedly connecting with the intermediate unit 31 at the position of the apex. In other embodiments, the connector 388a may be omitted.

The radial support force of the mesh support structure 32 of the embodiment can be achieved by adjusting the density, wire diameter, mesh shape, and size of the support wires in the first mesh region 37 and the second mesh region 38, and in addition, the mesh shape and size in the first mesh region 37 can be the same or different, and the mesh shape and size in the second mesh region 38 can be the same or different.

It can be appreciated that in other embodiments, the wave troughs 385 of the first hooking member 382 and the wave peaks 386 of the second hooking member 383 of some or all the hooking units 381 do not hook on each other, but are substantially, in an axial direction, separated from each other to form a gap, or may abut, but not hook onto each other. The above-mentioned non-hooking abutment includes the wave trough 385 of the first hooking member 382 and the wave peak 386 of the second hooking member 383 abut to each other substantially in the axial direction, or the first hooking member 382 and the second hooking member 383 overlaps each other in a partial region in the radial direction. The hooking units 381 that are not mutually hooked facilitate further flexibility of the second mesh region 38, and further facilitate axial expansion of the hooking units 381 connected thereto by the first mesh region 37 (e.g. the first hooking member 382 moving proximally and the second hooking member 383 moving distally), thereby allowing greater mesh expansion in the second mesh region 38 and facilitating the passage of the bridging stent through the second mesh region 38.

### Embodiment 2

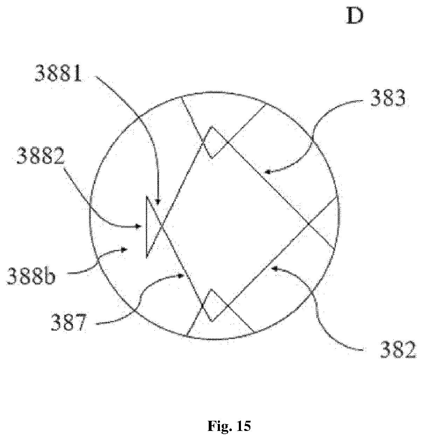

Referring to Fig. 14, the embodiment is substantially the same as the lumen stent 100 in Embodiment 1, except that the shape of the connector 388b is different. Referring also to Fig. 15, the connectors 388b of the embodiment are generally triangular in shape and are spaced apart in the axial direction. The connector 388b includes two waists 3881 and a bottom edge 3882 connecting the two waists 3881; the bottom edge 3882 extends substantially in an axial direction; one waist 3881 is formed by extending a wave rod 387 of a first hooking member 382, and the other waist 3881 is formed by extending a wave rod 387 of a second hooking member 383 which is adjacent to the first hooking member 382 and is not hooked thereon, and the intersection of the two waists 3881 of the connector 388b forms an apex at which the two waists 3881 can slide relatively. In other embodiments, the connector 388b may also be drop-shaped or any other suitable shape.

The intermediate unit 31 and the bottom edge 3882 of the connecting piece 388b can be connected through sewing to achieve a fixed connection between the intermediate unit 31 and the mesh support structure 32; the connecting piece 388b of the embodiment can facilitate the sewing operation, and can play a limiting role on the sewing thread to prevent the connecting piece 388b from sliding out of the sewing thread; in addition, the overlapping support wires at the apex of the connecting piece 388b can relatively slide, so that the flexibility of the edge of the mesh support structure 32 can be ensured, and the mesh size of the second mesh region 38 can be made variable, to facilitate the implantation of the bridging stent 3; in addition, when the mesh support structure 32 of the embodiment 1 is the same as the overall size of the embodiment, the waveform angle of the first hooking member 382 and the second hooking member 383 is made smaller due to the addition of the connector 388b to be more easily radially compressed, thereby facilitating assembly.

### Embodiment 3

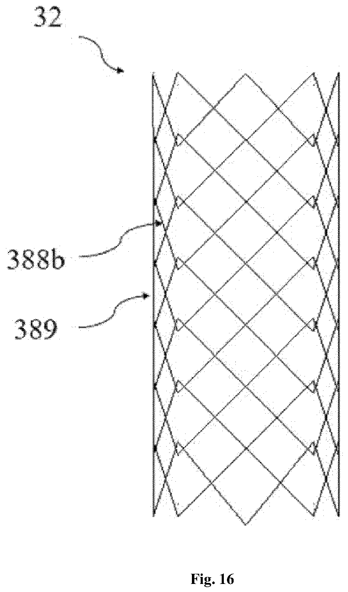

Referring to Fig. 16, the embodiment is substantially the same as the lumen stent 100 in Embodiment 1 and Embodiment 2, except that the mesh support structure 32 of the embodiment is further provided with support units 389 at both side edges in the circumferential direction, and the mesh support structure 32 is connected to the intermediate unit 31 through the support units 389 at both sides. The support unit 389 may be formed by hooking a plurality of connectors 388b as described in Embodiment 2 to each other in the axial direction, enhancing the supporting effect of the edge of the mesh support structure 32, and improving the stability of the form of the edge of the mesh support structure 32. In other embodiments, the support unit 389 includes a support rod (not shown) extending from the proximal end to the distal end and coupled to the edge of the second mesh region 38, which may be a unitary rod-like structure coupled to the edge of the second mesh region 38 by welding, bonding, etc. The support rod not only enhances the supporting effect of the edge of the mesh support structure 32 but also effectively prevents the mesh support structure 32 and the main body stent 1 from being shortened.

### Embodiment 4

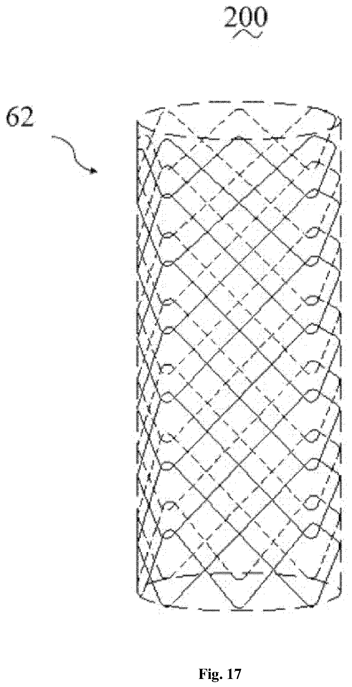

Referring to Fig. 17, the embodiment provides a lumen stent 200, the lumen stent 200 including a mesh support structure 62, the mesh support structure 62 having a generally hollow tubular shape with openings at both ends, including a first mesh region 37, and a second mesh region 38. The first mesh region 37 and the second mesh region 38 are spaced apart in the circumferential direction and are connected to each other.

The specific shapes and structures of the first mesh region 37 and the second mesh region 38 of the embodiment are the same as those of the embodiments 1-3 and will not be described in detail herein, except for the number and specific dimensional relationships of the first mesh region 37 and the second mesh region 38. In the embodiment, the mesh support structure 62 includes a plurality of first mesh regions 37 and a plurality of second mesh regions 38 (e.g. four first mesh regions 37 and four second mesh regions 38).

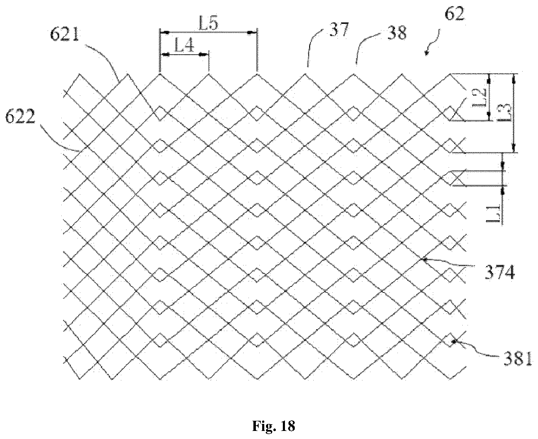

Referring also to Fig. 18, the mesh support structure 62 is formed by integral knitting, has a substantially circular cross-section, and includes a plurality of rows of first waveform units 621 and a plurality of rows of second waveform units 622; the wave troughs of the first waveform units 621 are hooked with the wave peaks of the second waveform units 622 to form hooking units 381, and the wave rods of the first waveform units 621 and the wave rods of the second waveform units 622 are overlapped with each other to form intersecting units 374. The inner diameter of the mesh support structure 62 is D1 and the hooking gap of the hooking unit 381 L1 ∈(0,1/9π×D1). The wave height of the first waveform unit 621 of the first row (e.g. most proximal) and the wave height of the second waveform unit 622 of the last row (e.g. most distal) are substantially equal, both L2. The wave heights of the other first waveform units 621 except for the first waveform unit 621 of the first row and the wave heights of the other second waveform units 622 except for the second waveform unit 622 of the last row are substantially equal, both being L3 E (2/3L2, 3L2), the circumferential distance between the wave peak of the first waveform unit 621 in the first row and the wave peak of the second waveform unit 622 adjacent thereto is L4, and the circumferential distance between the circumferentially adjacent hooking unit 381 is L5 E (1.5L4, 3L4). In other embodiments, each of the above dimensions may be adjusted according to actual needs.

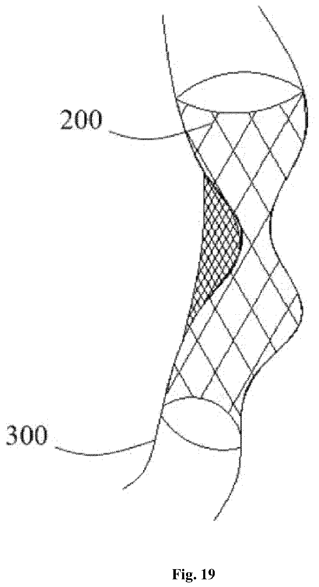

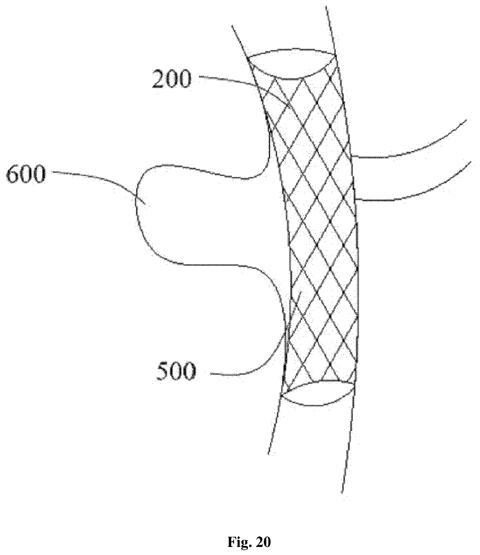

The lumen stent 200 of the embodiment may be a bare stent that is not covered, or a covered stent, and may be applied to a variety of scenarios. For example, as shown in Fig. 19, the lumen stent 200 of the embodiment can be used to prop up a stenotic vessel 300 to maintain patency of the lumen of the stenotic vessel 300, or to support a dissection to maintain the lumen space of the stenotic vessel 300. As shown in Fig. 20, this lumen stent 200 is implanted in the body as a part of the covered stent 500, to not only isolate the tumor body 600, repair the damaged artery, but also ensure the patency of blood flow in branches and reestablish the path of branches. The units in the first mesh region 37 are parallel and four-sided structures, with high plasticity, and facilitate the passage of the externally bridged stent through the units. The bare stent can alter lumen hemodynamics in the tumor, allowing less blood to enter the tumor body 600, accelerating the gradual thrombosis of the blood in the tumor. In addition, a specific effect can be achieved by setting the porosity of the mesh (the percentage of the area of all the meshes in the mesh support structure 62 to the area of the whole mesh support structure 62) and the mesh density, for example, by reducing the porosity of the mesh and increasing the mesh density, thereby effectively reducing the blood flow velocity in the tumor body 600. As another example, the mesh support structure 62 of the embodiment may be sleeved outside the intermediate unit 31 and connected at both ends to the proximal segment 10 and the distal segment 50, respectively, while a gap is formed between the outer surface of the intermediate unit 31 and the mesh support structure 62. The advantage of this arrangement is, at least, that the intermediate unit 31 is better protected from compression by the lumen inner wall to ensure patency and stability of the blood flow of the intermediate unit 31, and the bridging unit can be implanted from multiple directions and can be widely used in a variety of scenarios.

### Embodiment 5

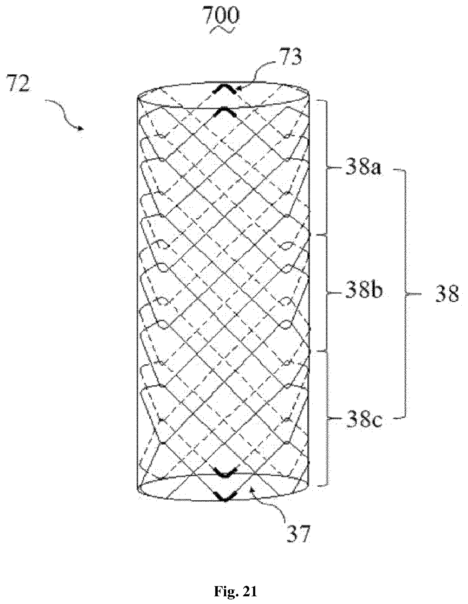

Referring to Fig. 21, the lumen stent 700 of the embodiment is substantially the same as that of the embodiment 4, except that the hooking gap L0 of the second mesh region 38 is different in the embodiment. The second mesh region 38 of the embodiment includes, in proximal to distal direction, a proximal region 38a, an intermediate region 38b, and a distal region 38c. In the naturally deployed state, all the hooking units 381 in the intermediate region 38b are in the first hooking state, and the hooking units 381 in the proximal region 38a and the distal region 38c are also in the first hooking state. The hooking gaps L0 of all the hooking units 381 in the intermediate region 38b are larger than the hooking gaps L0 of the hooking units 381 in the proximal region 38a and larger than the hooking gaps L0 of the hooking units 381 in the distal region 38c. Therefore, in the hooking unit 381 of the intermediate region 38b, there is more space for the first hooking member 382 and the second hooking member 383 to move toward both ends, respectively. When the side of the lumen stent 700 on which the mesh support structure 72 is positioned is bent outwardly, the intermediate region 38b may be stretched to a greater degree so that the lumen stent 700 may better conform to the bent lumen and better conform to the lumen inner wall. In addition, since the hooking gap L0 of the hooking unit 381 is smaller in the proximal region 38a and the distal region 38c, the first hooking member 382 and the second hooking member 383 in the hooking unit 381 are more restricted from each other, and the wave troughs 385 of the first hooking member 382 and the wave peaks 386 of the second hooking member 383 do not easily bow outward (or bow), thereby reducing irritation and damage to the inner wall of the lumen by the lumen stent 700. In other embodiments, the hooking gap L0 of a portion of the hooking unit 381 (e.g. at least one hooking unit 381) in the intermediate region 38b is greater than the hooking gap L0 of the hooking unit 381 in the proximal region 38a and greater than the hooking gap L0 of the hooking units 381 in the distal region 38c, so long as the intermediate region 38b can be stretched to a greater extent.

In addition, the lumen stent 700 of the embodiment is further provided with four development points 73 at the proximal and distal ends of the opposite two first mesh regions 37, respectively. The placement of the development point 73 facilitates more accurate intraoperative positioning by the operator.

### Embodiment 6

Referring to Fig. 21, the embodiment provides a lumen stent 800, the lumen stent 800 may be either a bare stent that is not covered or a covered stent, the lumen stent 800 includes a mesh support structure 82, the mesh support structure 82 is formed by integrally knitting, and which has a hollow both ends, including a first mesh region 37, a second mesh region 38, and a third mesh region 83. The mesh support structure 82 of the embodiment has two second mesh regions 38, each second mesh region 38 connecting the first mesh region 37 on one side and the third mesh region 83 on the other side in the circumferential direction. In other embodiments, the lumen stent 800 has two first mesh regions 37, a second mesh region 38, and a third mesh region 83, the second mesh region 38 being connected to one first mesh region 37 on both sides in the circumferential direction, and the third mesh region 83 also being connected to one first mesh region 37 on both sides in the circumferential direction. The first mesh region 37 and the second mesh region 38 have been described in detail in Embodiments 1 to 5, and will not be described in detail.

The third mesh region 83 of the embodiment includes one or more waveform unit groups 84 spaced axially apart, the waveform unit group 84 including two waveform units mutually hooked, a third waveform unit 841, and a fourth waveform unit 842, respectively. The wave troughs of the third waveform unit 841 and the wave peaks of the fourth waveform unit 842 hook with each other.

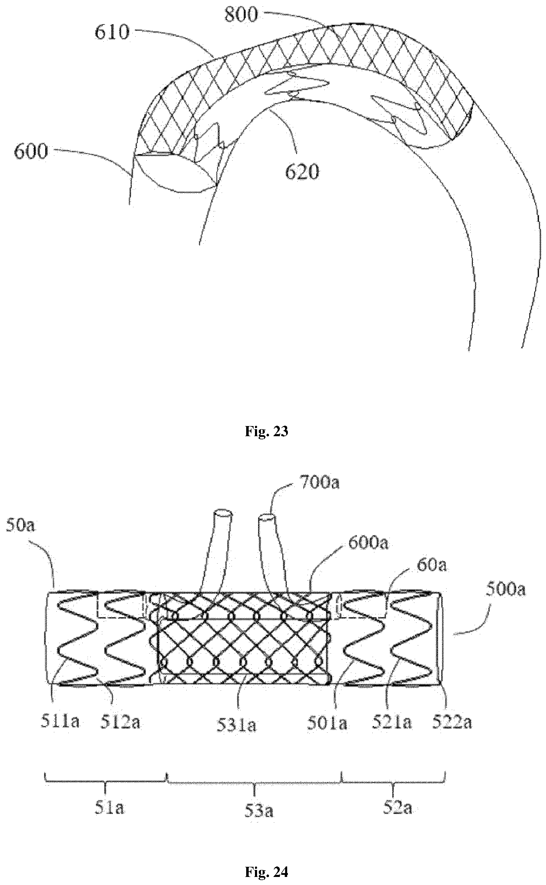

The third mesh region 83 has superior flexibility and can be easier to bend than the first mesh region 37 and the second mesh region 38. Referring to Fig. 23, when the target implantation lumen of the lumen stent 800 of the embodiment is an aortic arch 600 since the aortic arch 600 has a large curved side 610 (i.e. a side with a smaller degree of curvature and a larger radius of curvature) and a small curved side 620 (i.e. a side with a larger degree of curvature and a smaller radius of curvature), the first mesh region 37 and the second mesh region 38 can be oriented toward the large curved side 610 and the third mesh region 83 is oriented toward the small curved side 620 during implantation, so that the lumen stent 800 is more easily bent and better conforms to the curved shape of the lumen.

Further, the second mesh region 38 includes, in proximal to distal direction, a proximal region 38a, an intermediate region 38b, and a distal region 38c. In the naturally deployed state, all the hooking units 381 in the intermediate region 38b are in the first hooking state, and the hooking units 381 in the proximal region 38a and the distal region 38c are also in the first hooking state. The hooking gaps L0 of all the hooking units 381 in the intermediate region 38b are larger than the hooking gaps L0 of the hooking units 381 in the proximal region 38a and larger than the hooking gaps L0 of the hooking units 381 in the distal region 38c. Therefore, in the hooking unit 381 of the intermediate region 38b, there is more space for the first hooking member 382 and the second hooking member 383 to move toward both ends, respectively. When the side of the lumen stent 800 on which the mesh support structure 82 is positioned is bent outwardly, the intermediate region 38b may be stretched to a greater degree so that the lumen stent 800 may better conform to the bent lumen and better conform to the lumen inner wall. In addition, since the hooking gap L0 of the hooking unit 381 is smaller in the proximal region 38a and the distal region 38c, the first hooking member 382 and the second hooking member 383 in the hooking unit 381 are restrained from each other to a greater extent, and the wave troughs of the first hooking member 382 and the wave peaks of the second hooking member 383 are not easily tilted outward (or bowed), thereby reducing irritation and damage to the inner wall of the lumen by the lumen stent 800.

In the naturally expanded state, the waveform unit groups 84 of the third mesh region 83 of the embodiment are each in the first hooking state, and the hooking gap (not shown) formed between the wave troughs of the third waveform unit 841 and the wave peaks of the fourth waveform unit 842 is smaller than the hooking gap L0 of the hooking unit 381 in the second mesh region 38. In other embodiments, the two waveform units in the waveform unit group 84 of the third mesh region 83 may be in the second hooking state and/or the third hooking state, i.e. the hooking gap of the two waveform units in the waveform unit group 84 of the third mesh region 83 is zero, so that when the third mesh region 83 is located on the small curved side 620, the wave peaks and wave troughs of the waveform unit group 84 are not lifted outward due to the compression of the small curved side 620, thereby further reducing the stimulation and damage to the inner wall of the lumen by the lumen stent 800.

### Embodiment 7

As shown in Fig. 24, the embodiment provides a stent system including a covered stent 500a and a lumen stent 600a. The covered stent 500a has a hollow tubular structure with openings at both ends and includes a main body stent 50a and a branch stent 60a provided in the main body stent 50a; the inner cavity of the main body stent 50a communicates with the inner cavity of the branch stent 60a.

The main body stent 50a includes a main body support part and a main body covering film, and the main body covering film may be provided on an inner surface and/or an outer surface of the main body support part, or the main body covering film may be provided on a part of the inner surface and a part of the outer surface of the main body support part.

The main body stent 50a may be axially divided into a proximal segment 51a, a distal segment 52a, and an intermediate segment 53a between the proximal segment 51a and the distal segment 52a.

The proximal segment 51a includes a tubular proximal support part 511a and a proximal main body covering film 512a, the proximal main body covering film 512a may be applied to the inside and/or outside of the proximal support part 511a by sewing, bonding, heat-fusing, or the like. The proximal support part 511 includes a plurality of axially spaced main body wave coils 501a.

The distal segment 52a includes a tubular distal support part 511a and a distal main body covering film 522a, the distal main body covering film 522a may also be applied to the inside and/or outside of the distal support part 511a by stitching, adhesive, heat staking, or the like. The distal support part 511a includes a plurality of axially spaced main body wave coils 501a.

The intermediate segment 53a includes a tubular intermediate main body covering film 531a. One end of the intermediate main body covering film 531a is connected to the proximal segment 51a, and the other end is connected to the distal segment 52a, and the inner cavity formed by enclosing the intermediate main body covering film 531a is in communication with the inner cavity formed by enclosing the proximal main body covering film 512a and the inner cavity formed by enclosing the distal main body covering film 522a. In other embodiments, intermediate segment 53a may also include an intermediate support part (not shown), the intermediate support part is generally tubular and includes a plurality of axially spaced intermediate waveform units, and the intermediate waveform units are annular.

Alternatively, the intermediate waveform unit may have an arc shape that only partially covers the intermediate main body covering film 531a. The intermediate main body covering film 531a is applied to the inner side and/or the outer side of the intermediate support part by sewing, bonding, heat-fusing, or the like.

The lumen stent 600a is sleeved outside the intermediate segment 53, and the two ends of the lumen stent 600a are connected to the distal end of the proximal segment 51a and the proximal end of the distal segment 52a, respectively. At least a portion of the lumen stent 600a radially forms a gap (or void, cavity) with the outer surface of the intermediate segment 53a, which gap may communicate with the inner cavity of the branch stent 60a. For example, in the embodiment, a gap is formed between the lumen stent 600a and the outer surface of the intermediate segment 53a, i.e. in a natural state, any inner surface of the lumen stent 600a is not in contact with the outer surface of the intermediate segment 53a. The specific shape and structure of the lumen stent 600a can be described concerning Embodiments 4-6, which will not be described in detail herein.

When it is necessary to implant a bridging stent 700a for a branch vessel, the bridging stent 700a can pass through the lumen stent 600a and communicate with the branch stent 60a; in the process of reconstructing the branch, blood flow can enter the gap between the lumen stent 600a and the intermediate segment 53a through the branch stent 60a, and then enter the branch vessel from the lumen stent 600a without a covering film, to avoid the problem of long-term ischemia in the process of reconstructing the branch of the branch vessel; for twisted lumens, such as lumens having a small true lumen, by providing a gap between the lumen support 600a and the intermediate segment 53a to provide a more stable operation space during the operation, compression of the operation space due to the compression of the intermediate segment 53a by the lumen can be avoided.

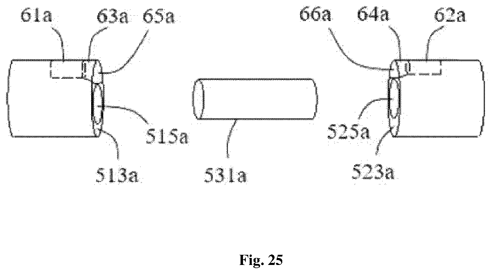

Referring to Fig. 25, the branch stent 60a includes a branch support part and a tubular branch covering film (not shown). The branch covering film may be applied to the inside and/or outside of the branch support part by sewing, bonding, heat staking, etc.

Branch stent 60a includes a proximal tube body 61a disposed within proximal segment 51a, and a distal tube body 62a disposed within distal segment 52a. The proximal tube body 61a communicates with the inner cavity of the proximal segment 51a and with the gap formed by the outer surface of the intermediate segment 53a and the lumen stent 600a. The distal tube body 62a communicates with the inner cavity of the distal segment 52a and communicates with the outer surface of the intermediate segment 53a and the gap formed by the lumen stent 600a. The proximal tube body 61a and the distal tube body 62a may be provided with only 0 or more pieces, respectively. For example, when it is applied to the aortic arch, two proximal tube bodies 61a and one distal tube body 62a may be provided; alternatively, one proximal tube body 61a and two distal tube bodies 62a may be provided, or all of them may be provided at the proximal or distal the distal end, which may be correspondingly provided and selected according to the number and position of the bridging stent 700a to be accessed so that it can meet the needs of emergency surgery in clinical use. The presence or absence and the number of the proximal tube body 61a and the distal tube body 62a can be correspondingly set according to the actual situations that may occur, to design a stent system with various specifications for optimal selection in a clinical emergency, and thus the specific embodiments thereof are not limited to the above-mentioned exemplary embodiments.

In the embodiment, proximal tube body 61a communicates with the gap through proximal transition segment 63a, and distal tube body 62a communicates with the gap through distal transition segment 64a. The proximal end of the proximal transition segment 63a has a proximal outer opening 65a communicating with the gap, the proximal end of the distal transition segment 64a has a distal outer opening 66a communicating with the gap, and the proximal outer opening 65a of the proximal transition segment 63a is disposed opposite the distal outer opening 66a of the distal transition segment 64a. Both the proximal transition segment 63a and the distal transition segment 64a may be flared with the reduced end of the flared proximal transition segment 63a facing proximally and the reduced end of the flared distal transition segment 64a facing distally. During the procedure, the bridging stent 700a may be accessed through the proximal outer opening 65a and the distal outer opening 66a. The enlarged ends of the proximal transition segment 63a and the distal transition segment 64a are both towards the intermediate segment 53a, such as towards the lumen stent 600a, and gradually contract in a direction away from the lumen stent 600a, to provide a relatively large implantation guiding path for the bridging stent 700a in the initial stage of implantation, to facilitate the rapid implantation of the bridging stent 700a, and gradually contract the implantation path thereof, thereby facilitating the more accurate and stable implantation of the bridging stent 700a; after the implantation of bridging stent 700a into the branch stent 60a, since the lumen stent 600a fixes it to a certain extent, it is possible to prevent the bridging stent 700a from the blood flow, so that the bridging stent 700a is relatively more stable.

The intermediate main body covering film 531a may be connected at one end to proximal segment 51a by proximal sealing film 513a and at the other end to distal segment 52a by distal sealing film 523a. The proximal sealing film 513a and the distal sealing film 523a are both of a sheet-like structure, a part of the edge of the proximal sealing film 513a is connected to the edge of the proximal outer opening 65a, and the remaining part is connected to the edge of the distal end of the proximal segment 51a; a portion of the edge of the distal sealing film 523a is connected to the edge of the distal outer opening 66a, and the remaining portion is connected to the edge of the proximal end of the distal segment 52a; the proximal sealing film 513a is provided with a first through-opening 515a; the first through-opening 515a enables the inner cavity of the intermediate main body covering film 531a to communicate with the inner cavity of the proximal segment 51a, and the distal sealing film 523a is provided with a second through-opening 525a; the second through-opening 525a enables the inner cavity of the intermediate main body covering film 531a to communicate with the inner cavity of the distal segment 52a. It can be appreciated that proximal sealing film 513a and distal sealing film 523a may be beveled so that there is a natural transition between proximal segment 51a and intermediate segment 53a, reducing the effect on blood flow.

In the embodiment, the diameter of the lumen stent 600a may be greater than or equal to the diameter of the proximal segment 51a and the distal segment 52a. The diameter of the lumen stent 600a is also only greater than or equal to the diameter of any one of the proximal segment 51a and the distal segment 52a, and when the diameter of the lumen stent 600a is equal to the diameter of the proximal segment 51a and/or the distal segment 52a when the intermediate segment 53a is compressed, the intermediate main body covering film 531a is not substantially compressed due to the support of the lumen stent 600a, ensuring that the main body blood flow is unobstructed. When the diameter of the lumen stent 600a is larger than the diameter of the proximal segment 51a and/or the distal segment 52a, the lumen stent 600a will be subjected to the radial pressure at first after implantation of the target vessel, and the radial pressure withstood is larger than the radial pressure of the proximal segment 51a and the distal segment 52a, to provide the main stent 10a with more stable and stronger radial supporting force, and thus have better anti-displacement performance. For example, in the embodiment, the ratio of the diameter of the lumen stent 600a to the diameter of the proximal segment 51a may be set to range from 1 to 1.2. At this time, the lumen stent 600a can not only provide a large radial supporting force but also provide a larger operation space for the implantation of the bridging stent 700a. It can be appreciated that in other embodiments, the diameter of the lumen stent 600a may also be smaller than the diameter of the proximal segment 51a, so long as the lumen stent 600a provides good support for the intermediate segment 53a and sufficient operating space for implantation of the bridging stent 700a.

In the embodiment, the ratio of the diameter of the intermediate segment 53a to the diameter of the lumen stent 600a is in the range of 0.5 to 0.9 to ensure that the intermediate segment 53a is not compressed and to facilitate the implantation of the bridging stent 700a. The intermediate segment 53a may be disposed of coaxially with the lumen stent 600a or may be disposed of on a different axis. In the embodiment, the central axis of the lumen stent 600a is closer to the branch stent 60a than the central axis of the intermediate segment 53a, providing a relatively large operating space for implantation of the bridging stent 700a.

In the embodiment, the proximal end of the lumen stent 600a is fixed to the inner side or outer side of the distal end of the proximal main body covering film 512a by sewing, bonding, etc. The distal end of the lumen stent 600a is fixed to the inner side or outer side of the proximal end of the distal main body covering film 522a, and the ratio of the axial length of the portion of the lumen stent 600a fixed to the proximal main body covering film 512a and the distal main body covering film 522a occupying the exposed portion of the lumen stent 600a (the portion of the lumen stent 600a not covering the covering film) is 0.1 to 1, so that the fixation of the lumen stent 600a is more stable, thereby ensuring the structural stability of the main body stent 50a.

In other embodiments, the above-described branch stent 60a may be omitted, and the intermediate segment 53a may be fenestrated to provide one or more windows through which the bridging stent 700a communicates with the main body stent 50a. Alternatively, in other embodiments, the covered stent 500a may include both the branch stent 60a and one or more windows on the intermediate segment 53a. However, it should be noted that if the branch stent 60a is omitted, both ends of the intermediate segment 53a should be sealed and connected to the proximal main body covering film 512a and the distal main body covering film 522a as far as possible.

It is to be understood that the proximal support part 511a, the distal support part 511a, and the intermediate support part are all part of the main body support part, and may be the same or different in structure, shape, size, and material from which they are made. The proximal main body covering film 512a, the distal main body covering film 522a, and the intermediate main body covering film 531a described above are all part of a main body covering film, which may be a one-piece structure or a split-piece structure (e.g. may be formed by splicing multiple covering films). In addition, the thickness of the proximal main body covering film 512a, the distal main body covering film 522a, and the intermediate main body covering film 531a may be the same or different materials.

The specific embodiments described above are only some of the embodiments, they should not be considered as limiting, the description is not exhaustive of all the embodiments of the concepts, some features of the different embodiments described above can be replaced or combined with each other, and those skilled in the art can also make substitutions according to actual needs.

## Claims

1. A lumen stent, comprising:
a mesh support structure,
wherein the mesh support structure comprises a first mesh region and a second mesh region connected to the first mesh region in a circumferential direction in a naturally deployed state;
the first mesh region comprises a plurality of rows of intersecting units formed by a plurality of first direction support wires spaced apart and a plurality of second direction support wires spaced apart overlapping each other;
the second mesh region comprises at least one row of hooking units, each row of the hooking units comprising one or more hooking units arranged axially, and the hooking unit comprising a first hooking member and a second hooking member; and
the first hooking member and the second hooking member in at least portion of the hooking units are mutually hooked substantially in an axial direction, or the first hooking member and the second hooking member in at least portion of the hooking units are mutually separated substantially in the axial direction to form a gap, or the first hooking member and the second hooking member in at least portion of the hooking units abut, but do not hook.

2. The lumen stent according to claim 1, further comprising:
a main body stent,
and the main body stent comprises a proximal segment, a distal segment, and an intermediate segment positioned between the proximal segment and the distal segment, the intermediate segment comprising an intermediate unit and at least one mesh support structure, and at least a portion of the mesh support structure and an outer surface of the intermediate unit forming a gap in a radial direction; the main body stent has an inner cavity extending through the proximal segment, the distal segment, and the intermediate unit, the gap being in communication with the inner cavity.

3. The lumen stent according to claim 2, wherein the mesh support structure comprises two second mesh regions in the circumferential direction, the two second mesh regions connecting to two sides of the first mesh region, respectively; and
the mesh support structure is connected to the intermediate unit through the second mesh regions on two sides.

4. The lumen stent according to claim 2, wherein the first hooking member comprises a wave trough and two wave rods connected to the wave trough, and the second hooking member comprises a wave peak and two wave rods connected to the wave peak; and
the first hooking member and the second hooking member are mutually hooked such that the wave trough of the first hooking member and the wave peak of the second hooking member are relatively movable in the axial direction.

5. The lumen stent according to claim 4, wherein the mesh support structure further comprises at least one connector connected to the second mesh region, and the mesh support structure is connected to the intermediate unit through the connector.

6. The lumen stent according to claim 5, wherein the connector is connected to one first hooking member and one second hooking member, and the connector comprises a wave towards a circumferential direction; and
the wave shares one wave rod with the first hooking member and one wave rod with the second hooking member.

7. The lumen stent according to claim 5, wherein the connector is connected to one first hooking member and one second hooking member, and the connector comprises two waists and a bottom edge connecting the two waists; and
one waist is formed by extending one wave rod of the first hooking member, the other waist is formed by extending one wave rod of the second hooking member, and the two waists slidably move relative to each other at an intersection.

8. The lumen stent according to claim 3, further comprising at least one support unit, wherein the support unit is provided at a peripheral edge of the mesh support structure.

9. The lumen stent according to any one of claims 1 to 8, wherein the second mesh region comprises a proximal region, an intermediate region, and a distal region from a proximal end to a distal end; and
a hooking gap of at least one hooking unit in the intermediate region is greater than a hooking gap of a hooking unit in the proximal region and greater than a hooking gap of a hooking unit in the distal region.

10. The lumen stent according to any one of claims 1 to 8, wherein
a distal end of the first hooking member and a proximal end of the second hooking member are mutually hooked, and the distal end of the first hooking member and/or the proximal end of the second hooking member are bent inward.

11. A lumen stent, comprising:
a first mesh region and a second mesh region connected to the first mesh region in a circumferential direction in a naturally deployed state;
the first mesh region comprises a plurality of rows of intersecting units formed by a plurality of first direction support wires spaced apart and a plurality of second direction support wires spaced apart overlapping each other;
the second mesh region comprises at least one row of hooking units, each row of the hooking units comprising one or more hooking units arranged axially, and the hooking unit comprising a first hooking member and a second hooking member; and
the first hooking member and the second hooking member in at least portion of the hooking units are mutually hooked substantially in an axial direction, or the first hooking member and the second hooking member in at least portion of the hooking units are mutually separated substantially in the axial direction to form a gap, or the first hooking member and the second hooking member in at least portion of the hooking units abut, but do not hook;
wherein the lumen stent has a tubular shape.

12. The lumen stent according to claim 11, wherein the lumen stent comprises a first mesh region and two second mesh regions in the circumferential direction, and the two second mesh regions connecting two sides of the first mesh region, respectively.

13. The lumen stent according to claim 11, wherein the first direction support wire extends to a first side for connection with the second hooking member, and the first direction support wire extends to a second side for connection with the first hooking member, the second direction support wire extends to a first side for connection with the first hooking member, and the second direction support wire extends to a second side for connection with the second hooking member.

14. The lumen stent according to claim 10, wherein the first hooking member comprises a wave trough and two wave rods connected to the wave trough, and the second hooking member comprises a wave peak and two wave rods connected to the wave peak, and the first hooking member and the second hooking member in at least portion of the hooking units are mutually hooked such that the wave trough of the first hooking member and the wave peak of the second hooking member are relatively movable in the axial direction.

15. The lumen stent according to claim 10, further comprising a third mesh region in the circumferential direction;
two sides of the third mesh region are each connected to one second mesh region; or
two sides of the third mesh region are each connected to one first mesh region; or
two sides of the third mesh region are each connected to the first mesh region and the second mesh region, respectively.

16. The lumen stent according to claim 15, wherein the third mesh region comprises one or more waveform unit groups spaced apart in an axial direction, the waveform unit group comprising two waveform units mutually hooked in the axial direction.

17. The lumen stent according to claim 16, wherein at least one set of two waveform units mutually hooked in the axial direction in the third mesh region has a hooking gap smaller than a hooking gap of the hooking units in the second mesh region.

18. The lumen stent according to claim 10, wherein the second mesh region comprises a proximal region, an intermediate region, and a distal region from a proximal end to a distal end; and
a hooking gap of at least one hooking unit in the intermediate region is greater than a hooking gap of a hooking unit in the proximal region and greater than a hooking gap of a hooking unit in the distal region.

19. The lumen stent according to claim 10, wherein the distal end of the first hooking member and the proximal end of the second hooking member are mutually hooked, and the distal end of the first hooking member and/or the proximal end of the second hooking member are bent inward.

20. A stent system, comprising the lumen stent according to any one of claims 10 to 19 and a covered stent connected to the lumen stent, wherein the lumen stent is sleeved outside the covered stent, and the lumen stent forms a gap with an outer surface of the covered stent in a radial direction.
